(19) Europäisches Patentamt
European Patent Office
Office européen des brevets

(11) Veröffentlichungsnummer : **0 033 151**
**B1**

(12) # EUROPÄISCHE PATENTSCHRIFT

(45) Veröffentlichungstag der Patentschrift : **02.05.84**

(51) Int. Cl.³ : **C 07 D277/56**, C 07 D277/12, C 07 D277/06, A 61 K 31/425

(21) Anmeldenummer : **81100532.1**

(22) Anmeldetag : **24.01.81**

(54) **Hydroxyphenyl-thiazol, -thiazolin- und -thiazolidin-carbon-säuren, Verfahren zu ihrer Herstellung und diese Verbindungen zur Verwendung zur Beeinflussung des Kollagenstoffwechsels.**

(30) Priorität : **29.01.80 DE 3002989**

(43) Veröffentlichungstag der Anmeldung : **05.08.81 Patentblatt 81/31**

(45) Bekanntmachung des Hinweises auf die Patenterteilung : **02.05.84 Patentblatt 84/18**

(84) Benannte Vertragsstaaten : **AT BE CH DE FR GB IT LI NL**

(56) Entgegenhaltungen :
**DE-A- 2 245 560**
**FR-A- 2 062 210**
**FR-A- 2 247 243**
**GB-A- 584 981**
**US-A- 3 882 110**
**JOURNAL OF THE AMERICAN CHEMICAL SOCIETY, Band 65, Nr. 11, 13. November 1943, New York, US, E.H. HUNTRESS et al.: "2-Phenylthiazole-4,5-dicarboxylic acid derivatives", Seiten 2167-2169**
**CHEMICAL ABSTRACTS, Band 87, 1977, Seite 599, Zusammenfassung Nr. 117845s, Columbus, Ohio, US,**

(73) Patentinhaber : **HOECHST AKTIENGESELLSCHAFT**
**Postfach 80 03 20**
**D-6230 Frankfurt am Main 80 (DE)**

(72) Erfinder : **Draeger, Eberhard, Dr.**
**Johannesallee 28**
**D-6230 Frankfurt am Main 80 (DE)**
Erfinder : **Lübbers, Henning, Dr.**
**Spechtstrasse 17**
**D-6231 Schwalbach (DE)**

**EP 0 033 151 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Hydroxyphenyl-thiazol, -thiazolin- und -thiazolidin-carbonsäuren, Verfahren zu ihrer Herstellung und diese Verbindungen zur Verwendung zur Beeinflussung des Kollagenstoffwechsels

Bei einer Reihe von Krankheiten (z. B. Skleroderma, Keloidbildung, Hypertrophen Narben, Diabetes, Leberzirrhose, Arteriosklerose) wird Kollagen in abnormal großer Menge synthetisiert und extrazellulär deponiert. Diese Kollagenüberproduktion ist oft unmittelbar für die schwerwiegenden Folgen der Krankheit verantwortlich. Die Reduktion der deponierten Kollagenmenge durch Medikamente hat einen positiven Einfluß auf den Krankheitsverlauf. Es ist bekannt, daß eine Behandlung der mit einer übermäßigen Kollagensynthese verbundenen Krankheiten mittels sehr unterschiedlicher Medikamente versucht wird.

Als Beispiel sei die Pharmakotherapie von Skleroderma genannt. Nach G. Herbai, B. Blom, H. Boström, Acta Med. Scand. 201, 203-206 (1977) werden oder wurden folgende Substanzgruppen und Substanzen beim Menschen in der Therapie eingesetzt :

D-thyroxin
Hydralazin
Adrenal corticosteroide (Prednisolon)
D-penicillamin (Cuprimine(R))
Stilboestrol-Derivate (Antioestrogene), Cyclofenil
Niedermolekulares Dextran (Theomakrodex)
Immunosupressiva (Azathioprin, Imurel(R))
Chlorambucil (Leukeran(R))
Benzyl-penicillin-diethylamino-ethylester
Gestagen-hormone Norethisteron (Primolut-Nor(R))
Hydroxyprogesteron-capronat (Proluton-depo(R))
Kalium-p-aminobenzoat, Salicyclate, Dimethyl-sulfoxid EDTA
Phenoxybenzamin (Dibenzyline), Dipyridamol (Persantin)
Nicotinsäure procain

Eine befriedigende Therapie konnte mit diesen Mitteln jedoch nicht oder nur bedingt erzielt werden, da das Wirkprinzip häufig nur die Beeinflussung sekundärer, durch die übermäßige Kollagenablagerungen indirekt hervorgerufener Störungen in symptomatischer Weise erlaubte, oder, im Falle der hormonartigen Substanzen, eine zu tiefgreifende Veränderung des Stoffwechselgeschehens bewirkt wurde.

Andere Substanzen wiederum, wie Penicillamin, sind durch ihre Eingriffe in andere Stoffwechselwege so sehr durch Nebenwirkungen belastet, daß eine Langzeittherapie nicht vertretbar erscheint.

Eine sehr selektive Hemmung der Kollagenbiosynthese läßt sich durch Beeinflussung der kollagenspezifischen Hydroxylierungsreaktionen erreichen. In deren Verlauf wird protein-gebundenes Prolin oder Lysin durch die Enzyme Prolin- bzw. Lysinhydroxylase hydroxyliert. Wird diese Reaktion durch Inhibitoren unterbunden, so entsteht ein nicht funktionsfähiges, unterhydroxyliertes Kollagenmolekül, das von der Zelle nur in geringer Menge in den extrazellulären Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich insgesamt die Menge des extrazellulär abgelagerten Kollagens.

Aus der FR-A-20 62 210 ist 2-(2-Hydroxyphenyl) thiazol-4-carbonsäure sowie deren Verwendung als antimikrobielles und entzündungshemmendes Mittel bekannt.

Inhibitoren der Prolin- und Lysinhydroxylase sind Gegenstand der vorliegenden Erfindung.

Es wurde gefunden, daß Verbindungen der Formeln

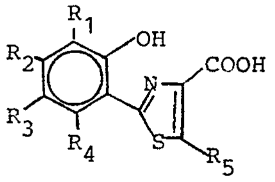

(I)

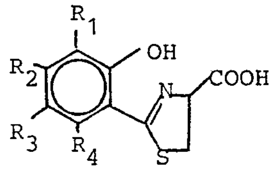

(II)

und

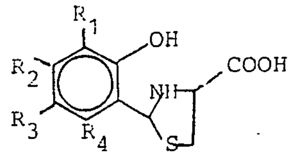

(III)

worin

$R_1$ und $R_3$ unabhängig voneinander Wasserstoff, Methyl, Chlor oder Brom sind und $R_1$ oder $R_3$ auch Nitro, Amino oder Sulfamoyl sein können,

$R_2$ Wasserstoff, Hydroxyl oder Methyl

$R_4$ Wasserstoff oder Methyl und

$R_5$ Wasserstoff, Methyl, Phenyl oder —COOH ist,

wobei 2-(2-Hydroxyphenyl)-thiazol-4-carbonsäure ausgenommen ist und wobei in Verbindungen der Formel III, falls $R_1$ oder $R_3$ nicht Amino oder Sulfamoyl sind, $R_4$ zusätzlich die Bedeutungen Chlor oder Brom haben kann, und deren Salze mit physiologisch verträglichen Basen selektiv wirksame Hemmstoffe der Kollagenbiosynthese sind.

Verbindungen der Formel III und ein Verfahren zu deren Herstellung sind beispielsweise aus J. Amer. Chem. Soc. *70* (1948) 1667 bekannt. Die oben genannten Verbindungen der Formeln I und II sind neu.

Bevorzugt sind Verbindungen der allgemeinen Formel I, in der $R_1$, $R_2$ und $R_4$ Wasserstoff und $R_3$ Methyl ist, in der $R_3$ oder $R_1$ und $R_3$ Chlor sind und die übrigen R Wasserstoff sind, in der $R_1$, $R_3$ und $R_4$ Wasserstoff und $R_2$ Hydroxyl sind und in der $R_1$ und $R_2$ Wasserstoff, $R_3$ Methyl und $R_4$ Phenyl oder COOH ist.

Weiter sind Verbindungen der Formel II bevorzugt, in der $R_1$ und $R_3$ unabhängig voneinander Wasserstoff, Methyl oder Chlor sind, $R_2$ Wasserstoff, Methyl oder Hydroxy und $R_4$ Wasserstoff oder Methyl sind.

Namentlich genannt seien insbesondere die folgenden bevorzugten erfindungsgemäßen Verbindungen der Formeln I, II oder III :

2-(5-Methyl-2-hydroxy-phenyl-)thiazol-4-carbonsäure-natrium
2-(5-Chlor-2-hydroxy-phenyl-)thiazol-4-carbonsäure-natrium
2-(5-Methyl-2-hydroxy-phenyl-)5-phenyl-thiazol-4-carbonsäure-natrium
2-(2,4-Dihydroxy-phenyl-)5-phenyl-thiazol-4-carbonsäure-natrium
2-(5-Methyl-2-hydroxy-phenyl-)thiazol-4,5-dicarbonsäure-natrium
2-(2-Hydroxy-phenyl-)thiazolin-4-carbonsäure-natrium
2-(5-Chlor-2-hydroxy-phenyl-)-thiazolin-4-carbonsäure-natrium
2-(2,4-Dihydroxy-phenyl-)thiazolin-4-carbonsäure-natrium
2-(2-Hydroxy-phenyl-)thiazolidin-4-carbonsäure
2-(3,5-Dichlor-2-hydroxy-phenyl-)thiazolidin-4-carbonsäure
2-(5-Nitro-2-hydroxy-phenyl-)thiazolidin-4-carbonsäure

Zur Salzbildung kann außer Natrium auch Kalium, Calzium oder Ammoniak herangezogen werden.

Die Carbonsäuren der allgemeinen Formel I können nach bekannten Methoden (Elderfield, Heterocyclic Compounds Bd V S. 624) aus Salicylsäurethioamiden durch Reaktion mit α-Halogencarbonylverbindungen nach folgender Gleichung hergestellt werden :

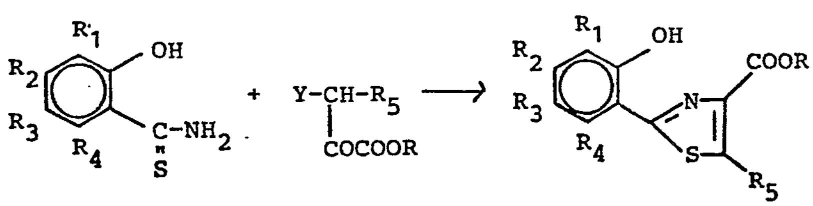

In der Gleichung haben $R_1$, $R_2$, $R_3$, $R_4$ die vorstehend angegebene Bedeutung, Y steht für Chlor oder Brom, R bedeutet Wasserstoff oder Alkyl, vorzugsweise solches mit 1-4 C-Atomen, $R_5$ kann Wasserstoff, Methyl, Phenyl oder —COOR sein. Im Falle R = Alkyl kann man die Ester, vorzugsweise alkalisch, zu I hydrolysieren.

Als α-Halogen Carbonylverbindungen können beispielsweise eingesetzt werden : Brombrenztraubensäure oder deren Ester, Phenylbrombrenztraubensäure oder deren Ester, Chlor- oder Brom-oxalessigester, 3-Brom-2-oxo-buttersäure oder deren Ester.

Bevorzugte Thioamide sind Salicylsäurethioamid, 5-Methyl-2-hydroxythiobenzamid oder 2,4 Dihydroxythiobenzamid.

Die Carbonsäuren der allgemeinen Formel II sind ebenfalls nach literaturbekannten Methoden zugänglich (Elderfield, Heterocyclic Compounds Bd V S. 681). Thioamide und Iminoäther reagieren mit Cystein oder seinen Derivaten zu Thiazolin-4-carbonsäure bzw. deren Derivaten. Dieses Verfahren ist auch auf Salicylsäurethioamide anwendbar.

Einen besonders glatten Reaktionsverlauf erreicht man jedoch erfindungsgemäß mit den O,N-Carbonylverbindungen der Salicylsäurethioamide, den 2-Oxo-4-thiono-dihydro-1,3-benzoxazinen, entsprechend folgender Gleichung :

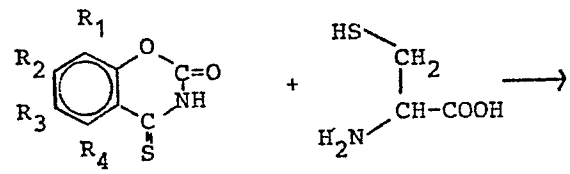

(II)

Die als Ausgangsmaterial dienenden Benzoxazinderivate können nach literaturbekannten Methoden (Pharmazie 21 (3) S. 161 (1966)) aus Salicylsäurethioamiden hergestellt werden. Bevorzugte Thioamide sind die gleichen wie oben angegeben.

Neben Cystein (D- oder L-Form oder Racemat) oder dessen Salzen wie dem Chlorhydrat lassen sich als Reaktionspartner auch Ester des Cysteins einsetzen.

Die Carbonsäuren der allgemeinen Formel III sind aus Salicylaldehyd oder seinen Derivaten durch Kondensation mit Cystein leicht zugänglich. Literaturangaben über die Kondensation von Carbonylverbindungen mit Cystein finden sich in Elderfield, Heterocyclic Compounds Bd V S. 698.

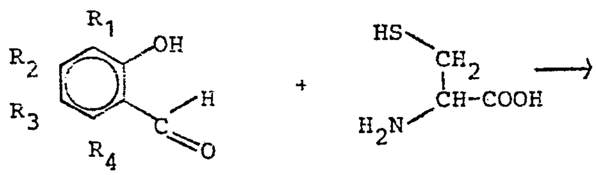

(III)

Bevorzugte Aldehyde sind Salicylaldehyd, 3,5-Dichlorsalicylaldehyd, 3,5-Dibromsalicylaldehyd oder 5-Nitrosalicylaldehyd.

Die Hemmwirkung der erfindungsgemäßen Substanzen auf die Kollagen-Biosynthese kann in einem Enzymtest analog zu der Methode von B. Peterkofsky und R. DiBlasio (Anal. Biochem. 66, 279-286 (1975)) überprüft werden. In diesem Test wird unterhydroxyliertes Kollagen in Gegenwart von molekularem Sauerstoff, Eisen-II-Ionen, α-Ketoglutarsäure und Ascorbinsäure enzymatisch hydroxyliert.

Als Enzyme können die Prolylhydroxylase oder Lysylhydroxylase in einem zellfreien Testmedium benutzt werden. Außerdem kann die Wirkung der Substanzen auf die Hydroxylierungsreaktion auch in der Zell- und Gewebekultur gemessen werden.

Die Hemmwirkung ($DI_{50}$) wird durch die Konzentration gekennzeichnet, bei der die Enzymreaktion zu 50 % gehemmt ist. Die $DI_{50}$-Werte einiger der erfindungsgemäßen Verbindungen sind in der folgenden Tabelle aufgeführt.

Tabelle

| Substanz | $DI_{50}$ Prolyl-hydroxylase (μM) | $DI_{50}$ Lysylhydroxy-lase (μM) | $DI_{50}$ Zellkultur (μM) |
|---|---|---|---|
| 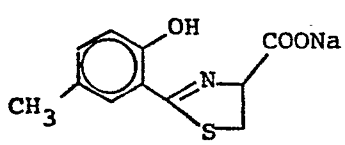 | 28 | 28 | 34 |
| 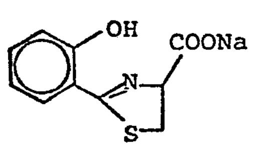 | 20 | 28 | |
| 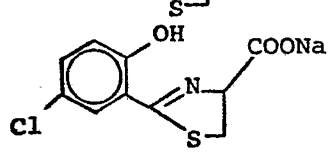 | 16 | 12 | 17 |

Tabelle (Fortsetzung)

| Substanz | $DI_{50}$ Prolyl-hydroxylase (µM) | $DI_{50}$ Lysylhydroxy-lase (µM) | $DI_{50}$ Zellkultur (µM) |
|---|---|---|---|
| 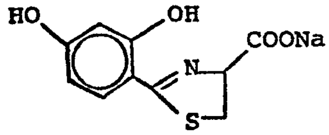 | 26 | 16 | |
| 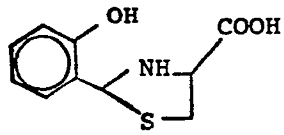 | 28 | 28 | |
| 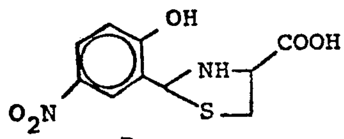 | 50 - 100 | 50 - 100 | |
| 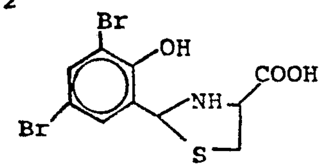 | 50 - 100 | 10 | |
| 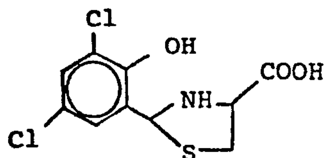 | 50 - 100 | 50 - 100 | |
| 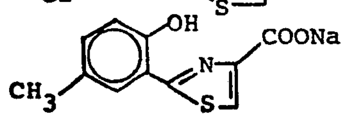 | 42 | 67 | |

| Substanz | $DI_{50}$ Lysylhydroxylase | µM Prolylhydroxylase Ⓟ |
|---|---|---|
| 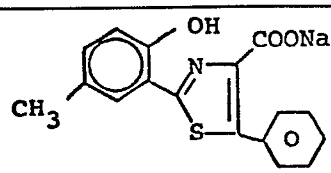 | 34 | 26 |
| 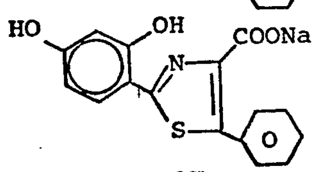 | 21 | 27 |
| 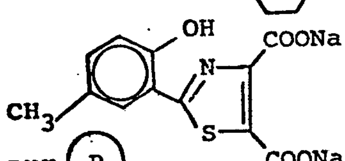 nur Ⓟ | 144 | 67 |
| 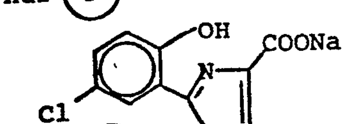 nur Ⓟ | 188 | 71 |
| 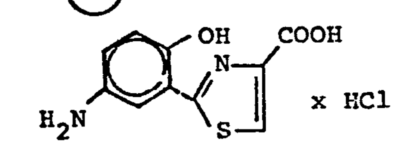 x HCl | 29 | 5,6 |

Die Erfindung wird an folgenden Beispielen erläutert.

Beispiel 1

Man gibt 7 g Phenylbrombrenztraubensäure, gelöst in 20 ml Eisessig, zu einer 60 °C warmen Lösung von 5 g 5-Methyl-2-hydroxy-thiobenzamid in 30 ml Eisessig. Das Reaktionsprodukt fällt sofort aus. Man verdünnt mit 25 ml Wasser, kocht auf und klärt heiß. Aus dem Filtrat kristallisieren 7,5 g (84 %) 2-(5-Methyl-2-hydroxy-phenyl-)5-phenyl-thiazol-4-carbonsäure vom Schmelzpunkt 193-194 °C. Durch Lösen in der berechneten Menge Sodalösung erhält man das Natriumsalz dieser Verbindung.

Beispiel 2

Analog Beispiel 1 erhält man aus Phenylbrombrenztraubensäure und 2,4 Dihydroxy-thiobenzamid die 2-(2,4-Dihydroxy-phenyl-)5-phenyl-thiazol-4-carbonsäure vom Schmelzpunkt 335-340°. Durch Lösen in $Na_2CO_3$-Lösung und Zugabe von Natriumchlorid gewinnt man das Natriumsalz.

Beispiel 3

Durch Kondensation molarer Mengen von 5-Methyl-2-hydroxythiobenzamid und Chlor-oxalessigsäureäureäthylester erhält man den 2-(5-Methyl-2-hydroxy-phenyl-)thiazol-4,5-dicarbonsäureäthylester vom Schmelzpunkt 80-81°. Kocht man diesen Ester in alkoholischer Natronlauge so gelangt man direkt zum Di-natriumsalz der 2-(5-Methyl-2-hydroxy-phenyl-)thiazol-4,5-dicarbonsäure. (Schmelzpunkt der Säure : 264-266°).

Beispiel 4

20 g Brenztraubensäure bromiert man in 30 ml Wasser mit 35 g Brom bei 70-80°. In die erhaltene Lösung von Brombrenztraubensäure trägt man bei 20° 28 g 5-Chlor-2-hydroxythiobenzamid ein. Die Temperatur steigt auf 45° und das Reaktionsprodukt kristallisiert allmählich aus. Man kühlt, verdünnt mit 100 ml Äthanol, saugt ab und erhält nach dem Trocknen 21 g 2-(5-Chlor-2-hydroxy-phenyl)-thiazol-4-carbonsäure vom Schmelzpunkt 292-294°. Durch Lösen in Sodalösung in der Wärme erhält man beim Abkühlen das Natriumsalz.

Beispiel 5

Analog Beispiel 4 erhält man aus 5-Methyl-2-hydroxy-phenylthiobenzamid und Brombrenztraubensäure die 2-(5-Methyl-2-hydroxy-phenyl)-thiazol-4-carbonsäure vom Schmelzpunkt 273-274°.

Beispiel 6

40 g Cysteinhydrochlorid werden in 150 ml Sauerstoff-freiem Wasser gelöst, 40 g 6-Methyl-2-oxo-4-thion-dihydro-1,3-benzoxazin (hergestellt nach Einhorn u. Mettler (B *35,* 350 (1902)) aus 5-Methyl-2-hydroxy-thiobenzoesäureamid) zugegeben und dann unter langsamem Durchleiten von Stickstoff 7,5 ml 33 %ige NaOH zugetropft. Man erwärmt auf 80-90° und tropft während 3 Std. weitere 12,5 ml konzentrierte NaOH zu. Nach weiteren 3 Std. fügt man 50 g Natriumchlorid zu und läßt unter Abkühlen auskristallisieren. Nach Umkristallisation aus wenig Wasser erhält man 35 g 2-(5-Methyl-2-hydroxy-phenyl)-thiazolin-4-carbonsäure-natrium. Die freie Carbonsäure schmilzt bei 255°.

Auf die gleiche Weise erhält man die folgenden Thiazolin-derivate :

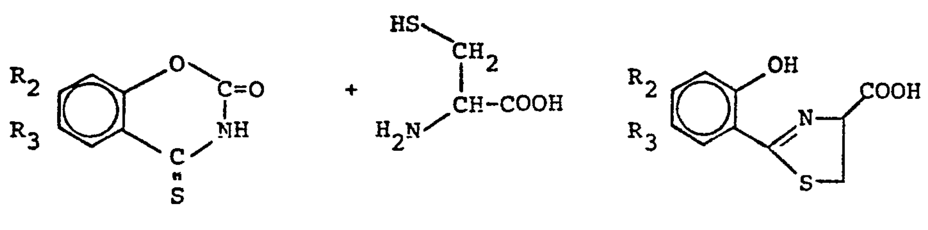

| Beispiel | $R_2$ | $R_3$ | Schmelzpunkt |
|---|---|---|---|
| 7 | H | H | 260° |
| 8 | H | Cl | 282° |
| 9 | $CH_3$ | $CH_3$ | 261/2° |
| 10 | OH | H | 275/80° |

Beispiel 11

20 g L(+) Cystein-hydrochlorid werden in 150 ml Wasser gelöst, 12 g Kaliumacetat und danach 100 ml Äthanol zugegeben. Man vermischt mit einer Lösung von 17 g Salicylaldehyd in 50 ml Äthanol. Die Temperatur steigt auf 35° und das Reaktionsprodukt kristallisiert nach kurzer Zeit aus. Man erhält 19 g 2-(2-Hydroxyphenyl)-thiazolidin-4-carbonsäure vom Schmelzpunkt 169° und einem $\alpha_D^{24}$ von − 44,5°.

In gleicher Weise erhält man die folgenden Thiazolidine :

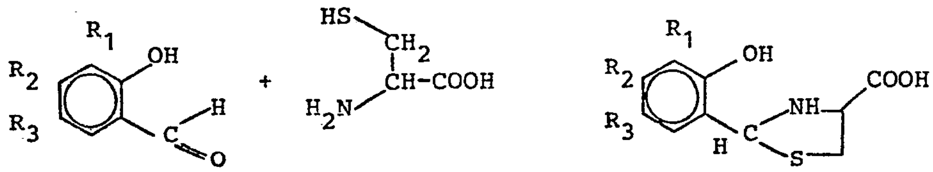

| Beispiel | $R_1$ | $R_2$ | $R_3$ | Schmelzpunkt | $\alpha_D$ |
|---|---|---|---|---|---|
| 12 | Cl | H | Cl | 165/6° | -48,9° |
| 13 | Br | H | Br | 167/8° | -98,3° |
| 14 | H | H | $NO_2$ | 158/60° | -40,1° |

**Ansprüche** (für die Vertragsstaaten : BE, CH, DE, FR, GB, IT, LI, NL)

1. Carbonsäure der Formel

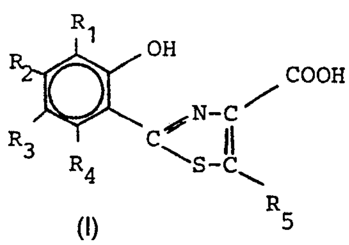

(I)

oder

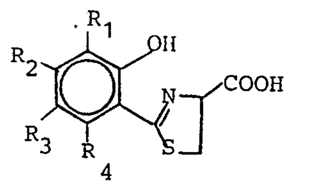

(II)

in der

$R_1$ und $R_3$ unabhängig voneinander Wasserstoff, Methyl, Chlor oder Brom sind und $R_1$ oder $R_3$ außerdem Nitro, Amino oder Sulfamoyl sein kann,

$R_2$ Wasserstoff, Hydroxyl oder Methyl,

$R_4$ Wasserstoff oder Methyl und

$R_5$ Wasserstoff, Methyl, Phenyl oder COOH ist,

wobei 2-(2-Hydroxyphenyl)-thiazol-4-carbonsäure ausgenommen ist, und deren Salze mit physiologisch verträglichen Basen.

2. Carbonsäure der Formel I nach Anspruch 1 in der $R_1$, $R_2$ und $R_4$ Wasserstoff und $R_3$ Methyl ist.

3. Carbonsäure der Formel I nach Anspruch 1 in der $R_3$ oder $R_1$ und $R_3$ Chlor ist und die übrigen R Wasserstoff sind.

4. Carbonsäure der Formel I nach Anspruch 1 in der $R_1$, $R_3$ und $R_4$ Wasserstoff und $R_2$ Hydroxyl ist.

5. Carbonsäure der Formel I nach Anspruch 1 in der $R_1$ und $R_2$ Wasserstoff, $R_3$ Methyl und $R_5$ Phenyl oder COOH ist.

6. Carbonsäure der Formel II gemäß Anspruch 1 in der $R_1$ und $R_3$ unabhängig voneinander Wasserstoff, Methyl oder Chlor sind, $R_2$ Wasserstoff, Methyl oder Hydroxyl und $R_4$ Wasserstoff oder Methyl ist und deren Salze mit physiologischen Basen.

7. Carbonsäure der Formel II nach Anspruch 1 in der $R_1$, $R_2$, $R_3$, $R_4$ Wasserstoff oder $R_1$, $R_2$, $R_4$ Wasserstoff und $R_3$ Methyl oder $R_1$, $R_4$ Wasserstoff und $R_2$, $R_3$ Methyl sind.

8. Carbonsäure der Formel II nach Anspruch 1 in der $R_1$, $R_2$, $R_4$ Wasserstoff und $R_3$ Chlor ist.

9. Carbonsäure der Formel II nach Anspruch 1 in der $R_1$, $R_3$, $R_4$ Wasserstoff und $R_2$ Hydroxyl ist.

10. Natriumsalz einer Carbonsäure nach Ansprüchen 1 bis 9.

11. Verfahren zur Herstellung von Carbonsäuren gemäß Anspruch 1, dadurch gekennzeichnet, daß man ein Salizylthioamid mit einer α-Halogen-carbonylverbindung der Formel

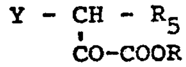

in der Y Chlor oder Brom, $R_5$ Wasserstoff, Methyl, Phenyl oder —COOR und R Wasserstoff oder Alkyl bedeutet oder mit Cystein oder einem Cystein-Salz oder -Ester umsetzt.

12. Verfahren zur Herstellung einer Carbonsäure der Formel II gemäß Anspruch 1, dadurch gekennzeichnet, daß man eine Verbindung der Formel

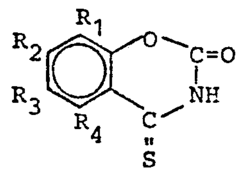

mit Cystein oder einem seiner Salze umsetzt.

13. Mittel enthaltend eine Carbonsäure gemäß Ansprüchen 1-10 oder deren Salze.

14. Verbindung gemäß einem der Ansprüche 1 bis 10 zur Verwendung als Mittel zur Beeinflussung des Kollagenstoffwechsels.

15. Carbonsäure der Formel III

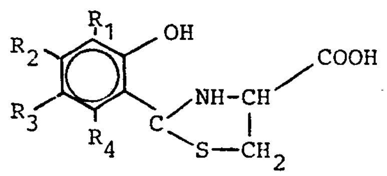

(III)

in der $R_1$, $R_3$ und $R_4$ Wasserstoff, Methyl, Chlor oder Brom sind und $R_1$ oder $R_3$ außerdem Nitro sein kann und $R_2$ Wasserstoff, Methyl oder Hydroxyl ist oder deren Salz mit einer physiologisch verträglichen Base als Mittel zur Beeinflussung des Kollagenstoffwechsels.

16. Verbindung gemäß einem der Ansprüche 1 bis 10 und 15 zur Verwendung als Heilmittel zur Behandlung von Krankheiten, bei denen der Kollagenstoffwechsel pathologisch verändert ist.

**Ansprüche** (für den Vertragsstaat AT)

1. Verfahren zur Herstellung von Carbonsäuren der allgemeinen Formel

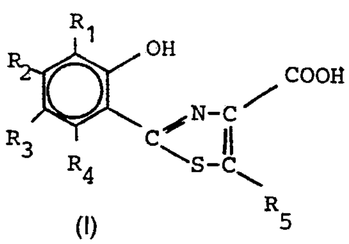

(I)

oder

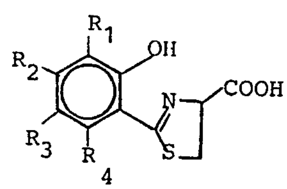

(II)

in der

$R_1$ und $R_3$ unabhängig voneinander Wasserstoff, Methyl, Chlor oder Brom sind und $R_1$ oder $R_3$ außerdem Nitro, Amino oder Sulfamoyl sein kann,

$R_2$ Wasserstoff, Hydroxyl oder Methyl,

$R_4$ Wasserstoff oder Methyl und

$R_5$ Wasserstoff, Methyl, Phenyl oder COOH ist,

wobei 2-(2-Hydroxyphenyl)-thiazol-4-carbonsäure ausgenommen ist, und deren Salzen mit physiologisch verträglichen Basen, dadurch gekennzeichnet, daß man ein Salizylthioamid mit einer α-Halogen-carbonylverbindung der Formel

$$Y - \underset{\displaystyle CO{-}COOR}{CH} - R_5$$

in der Y Chlor oder Brom, $R_5$ Wasserstoff, Methyl, Phenyl oder —COOR und R Wasserstoff oder Alkyl bedeutet oder mit Cystein oder einem Cystein-Salz oder -Ester umsetzt, und die erhaltene Verbindung gegebenenfalls in ein Salz überführt.

2. Verfahren zur Herstellung einer Carbonsäure der Formel II gemäß Anspruch 1, dadurch gekennzeichnet, daß man eine Verbindung der Formel

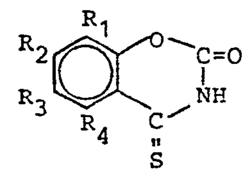

mit Cystein oder einem seiner Salze umsetzt, und die erhaltene Verbindung gegebenenfalls in ein Salz überführt.

3. Verfahren nach einem der Ansprüche 1 oder 2, dadurch gekennzeichnet, daß in der allgemeinen Formel I bzw. II $R_1$, $R_2$ und $R_4$ Wasserstoff und $R_3$ Methyl ist.

4. Verfahren nach einem der Ansprüche 1 oder 2, dadurch gekennzeichnet, daß in der allgemeinen Formel I bzw. II $R_3$ oder $R_1$ und $R_3$ Chlor ist und die übrigen R Wasserstoff sind.

5. Verfahren nach einem der Ansprüche 1 oder 2, dadurch gekennzeichnet, daß in der allgemeinen Formel I bzw. II $R_1$, $R_3$ und $R_4$ Wasserstoff und $R_2$ Hydroxyl ist.

6. Verfahren nach einem der Ansprüche 1 oder 2, dadurch gekennzeichnet, daß in der allgemeinen Formel I bzw. II $R_1$ und $R_2$ Wasserstoff, $R_3$ Methyl und $R_5$ Phenyl oder COOH ist.

7. Verfahren gemäß einem der Ansprüche 1 oder 2, dadurch gekennzeichnet, daß in der allgemeinen Formel II $R_1$ und $R_3$ unabhängig voneinander Wasserstoff, Methyl oder Chlor sind, $R_2$ Wasserstoff, Methyl oder Hydroxyl und $R_4$ Wasserstoff oder Methyl ist.

8. Verfahren nach einem der Ansprüche 1 oder 2, dadurch gekennzeichnet, daß in der allgemeinen Formel II $R_1$, $R_2$, $R_3$, $R_4$ Wasserstoff oder $R_1$, $R_2$, $R_4$ Wasserstoff und $R_3$ Methyl oder $R_1$, $R_4$ Wasserstoff und $R_2$, $R_3$ Methyl sind.

9. Verfahren nach einem der Ansprüche 1 oder 2, dadurch gekennzeichnet, daß in der allgemeinen Formel II $R_1$, $R_2$, $R_4$ Wasserstoff und $R_3$ Chlor ist.

10. Verfahren nach einem der Ansprüche 1 oder 2, dadurch gekennzeichnet, daß in der allgemeinen Formel II $R_1$, $R_3$, $R_4$ Wasserstoff und $R_2$ Hydroxyl ist.

11. Verfahren gemäß einem der Ansprüche 1 bis 10 zur Herstellung einer Verbindung zur Verwendung als Mittel zur Beeinflussung des Kollagenstoffwechsels.

12. Verfahren zur Herstellung einer Verbindung der Formel III

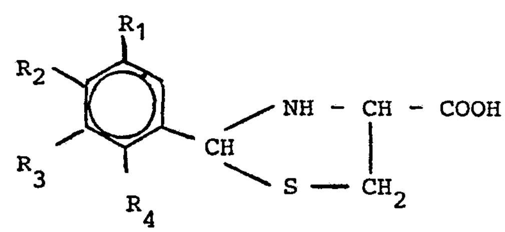

(III)

in der $R_1$, $R_3$ und $R_4$ Wasserstoff, Methyl, Chlor oder Brom sind und $R_1$ oder $R_3$ außerdem Nitro sein kann und $R_2$ Wasserstoff, Methyl oder Hydroxyl ist, zur Verwendung als Mittel zur Beeinflussung des Kollagenstoffwechsels, dadurch gekennzeichnet, daß man eine Verbindung der Formel

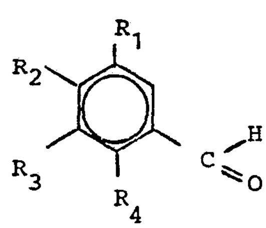

in der die Reste $R_1$-$R_4$ die vorstehend definierte Bedeutung haben, mit Cystein umsetzt.

**Claims** (for the Contracting States : BE, CH, DE, FR, GB, IT, LI, NL)

1. Carboxylic acid of the formula

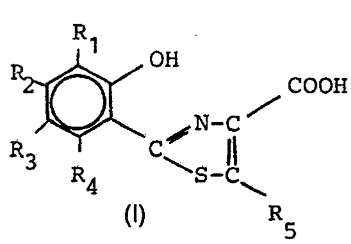

(I)

or

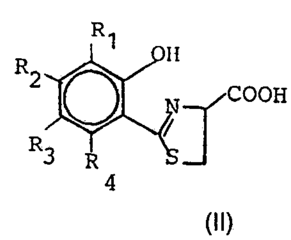

(II)

in which

$R_1$ and $R_3$, independently from each other, are hydrogen, methyl, chlorine or bromine, and $R_1$ or $R_3$ may alternatively be nitro, amino or sulfamoyl ;

$R_2$ is hydrogen, hydroxyl or methyl ;

$R_4$ is hydrogen or methyl ; and

$R_5$ is hydrogen, methyl, phenyl or COOH

with the exception of 2-(2-hydroxyphenyl)-thiazole-4-carboxylic acid, and their salts with physiologically acceptable bases.

2. Carboxylic acid of the formula I as claimed in Claim 1, wherein $R_1$, $R_2$ and $R_4$ are hydrogen and $R_3$ is methyl.

3. Carboxylic acid of the formula I as claimed in Claim 1, wherein $R_3$ or $R_1$ and $R_3$ are chlorine, and the other R are hydrogen.

4. Carboxylic acid of the formula I as claimed in Claim 1, wherein $R_1$, $R_3$ and $R_4$ are hydrogen and $R_2$ is hydroxyl.

5. Carboxylic acid of the formula I as claimed in Claim 1, wherein $R_1$ and $R_2$ are hydrogen, $R_3$ is methyl and $R_5$ phenyl or COOH.

6. Carboxylic acid of the formula II as claimed in Claim 1 wherein $R_1$ and $R_3$, independently from each other, are hydrogen, methyl or chlorine ; $R_2$ is hydrogen, methyl or hydroxyl ; and $R_4$ is hydrogen or methyl ; and their salts with physiologically acceptable bases.

7. Carboxylic acid of the formula II as claimed in Claim 1, wherein $R_1$ through $R_4$ are hydrogen or $R_1$, $R_2$, $R_4$ are hydrogen and $R_3$ is methyl, or $R_1$, $R_4$ are hydrogen and $R_2$, $R_3$ are methyl.

8. Carboxylic acid of the formula II as claimed in Claim 1, wherein $R_1$, $R_2$ and $R_4$ are hydrogen and $R_3$ is chlorine.

9. Carboxylic acid of the formula II as claimed in Claim 1, wherein $R_1$, $R_3$ and $R_4$ are hydrogen and $R_2$ is hydroxyl.

10. Sodium salt of a carboxylic acid as claimed in Claims 1 to 9.

11. A process for the preparation of carboxylic acids as claimed in Claim 1, which comprises reacting a salicylic thioamide with an alpha-halocarbonyl compound of the formula

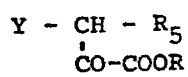

in which Y is chlorine or bromine, $R_5$ is hydrogen, methyl, phenyl or —COOR, and R is hydrogen or alkyl, or with a cysteine salt or cysteine ester.

12. A process for the preparation of a carboxylic acid of the formula II as claimed in Claim 1, which comprises reacting a compound of the formula

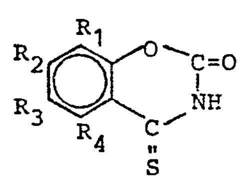

with cysteine or one of its salts.

13. Agent containing a carboxylic acid as claimed in Claims 1 to 10 or a salt thereof.

14. Carboxylic acid as claimed in Claims 1 to 10 for use as agent for influencing the collagen metabolism.

15. Carboxylic acid of the formula III

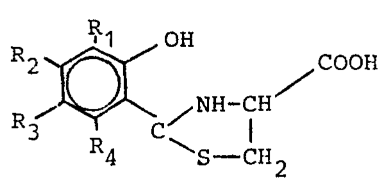

(III)

in which $R_1$, $R_3$ and $R_4$ are hydrogen, methyl, chlorine or bromine, and $R_1$ or $R_3$ may furthermore be nitro ; and $R_2$ is hydrogen, methyl or hydroxyl ; or of the salt thereof with a physiologically acceptable base for use as agent for influencing the collagen metabolism.

16. Carboxylic acid as claimed in Claims 1 to 10 and 15 for use as medicament for treating diseases with pathologically altered collagen metabolism.

**Claims** (for the Contracting State AT)

1. A process for the preparation of carboxylic acids of the general formula

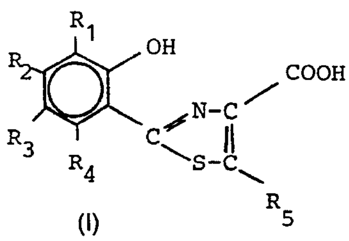 or 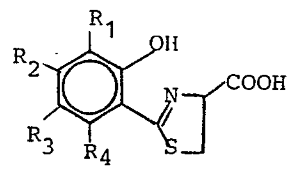

(I) (II)

in which

$R_1$ and $R_3$, independently from each other, are hydrogen, methyl, chlorine or bromine, and $R_1$ or $R_3$ may alternatively be nitro, amino or sulfamoyl ;

$R_2$ is hydrogen, hydroxyl or methyl ;

$R_4$ is hydrogen or methyl ; and

$R_5$ is hydrogen, methyl, phenyl or COOH,

with the exception of 2-(2-hydroxyphenyl)-thiazole-4-carboxylic acid, and their salts with physiologically acceptable bases, which comprises reacting a salicylic thioamide with an alpha-halocarbonyl compound of the formula

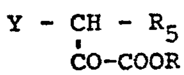

in which Y is chlorine or bromine, $R_5$ is hydrogen, methyl, phenyl or —COOR, and R is hydrogen or alkyl, or with a cysteine salt or cysteine ester, and optionally converting the compound thus obtained into a salt.

2. A process for the preparation of a carboxylic acid of the formula II according to Claim 1, which comprises reacting a compound of the formula

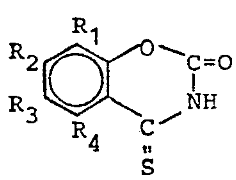

with cysteine or one of its salts, and converting the compound thus obtained into a salt.

3. A process as claimed in one of Claims 1 or 2, characterized in that in the general formula I and II, respectively $R_1$, $R_2$ and $R_4$ are hydrogen and $R_3$ is methyl.

4. A process as claimed in one of Claims 1 or 2, characterized in that in the general formula I and II, respectively $R_3$ or $R_1$ and $R_3$ are chlorine, and the other R are hydrogen.

5. A process as claimed in one of Claims 1 or 2, characterized in that in the general formula I and II, respectively, $R_1$, $R_3$ and $R_4$ are hydrogen and $R_2$ is hydroxyl.

6. A process as claimed in one of Claims 1 or 2, characterized in that in the general formula I and II, respectively, $R_1$ and $R_2$ are hydrogen, $R_3$ is methyl and $R_5$ phenyl or COOH.

7. A process as claimed in one of Claims 1 or 2, characterized in that in the general formula II $R_1$ and $R_3$, independently from each other, are hydrogen, methyl or chlorine; $R_2$ is hydrogen, methyl or hydroxyl ; and $R_4$ is hydrogen or methyl.

8. A process as claimed in one of Claims 1 or 2, characterized in that in the general formula II $R_1$ through $R_4$ are hydrogen or $R_1$, $R_2$, $R_4$ are hydrogen and $R_3$ is methyl, or $R_1$, $R_4$ are hydrogen and $R_2$, $R_3$ are methyl.

9. A process as claimed in one of Claims 1 or 2, characterized in that in the general formula II $R_1$, $R_2$ and $R_4$ are hydrogen and $R_3$ is chlorine.

10. A process as claimed in one of Claims 1 or 2, characterized in that in the general formula II $R_1$, $R_3$ and $R_4$ are hydrogen and $R_2$ is hydroxyl.

11. A process as claimed in anyone of Claims 1 to 10 for the preparation of a compound for use as agent for influencing the collagen metabolism.

12. A process for the preparation of a compound of the formula III

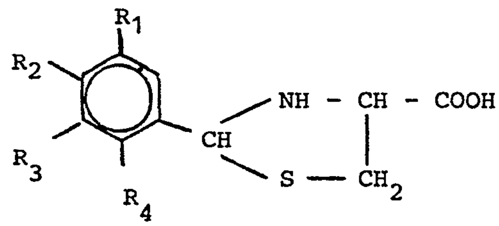

(III)

in which $R_1$, $R_3$ and $R_4$ are hydrogen, methyl, chlorine or bromine, and $R_1$ or $R_3$ may furthermore be nitro ; and $R_2$ is hydrogen, methyl or hydroxyl, for use as agent for influencing the collagen metabolism ; characterized in that a compound of the formula

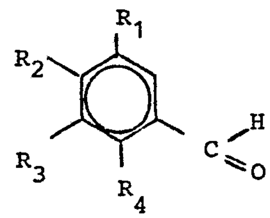

in which the residues $R_1$-$R_4$ are as defined above, is reacted with cysteine.

**Revendications** (pour les Etats contractants : BE, CH, DE, FR, GB, IT, LI, NL)

1. Acide carboxylique de formule

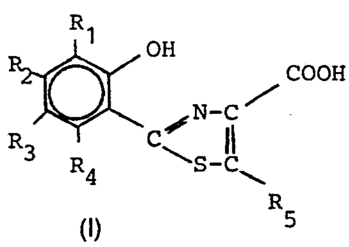

(I)

ou

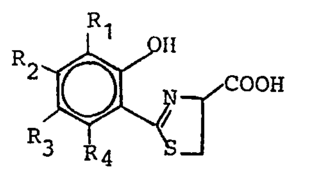

(II)

dans lesquelles

$R_1$ et $R_3$ représentent indépendamment l'un de l'autre l'hydrogène, un groupe méthyle, le chlore ou le brome et $R_1$ ou $R_3$ peuvent en outre représenter un groupe nitro, amino ou sulfamoyl,

$R_2$ représente l'hydrogène, un groupe hydroxyle ou méthyle,

$R_4$ représente l'hydrogène ou un groupe méthyle, et

$R_5$ représente l'hydrogène, un groupe méthyle, phényle ou —COOH,

l'acide 2-(2-hydroxyphényl)-thiazole-4-carboxylique étant exclus, et ses sels avec des bases physiologiquement acceptables.

2. Acide carboxylique de formule I selon la revendication 1, dans lequel $R_1$, $R_2$ et $R_4$ représentent l'hydrogène et $R_3$ est un groupe méthyle.

3. Acide carboxylique de formule I selon la revendication 1, dans lequel $R_3$ ou $R_1$ et $R_3$ représentent le chlore et les autres R représentent l'hydrogène.

4. Acide carboxylique de formule I selon la revendication 1, dans lequel $R_1$, $R_3$ et $R_4$ représentent l'hydrogène et $R_2$ est un groupe hydroxyle.

5. Acide carboxylique de formule I selon la revendication 1, dans lequel $R_1$ et $R_2$ représentent l'hydrogène, $R_3$ est un groupe méthyle et $R_5$ est un groupe phényle ou COOH.

6. Acide carboxylique de formule II selon la revendication 1, dans lequel $R_1$ et $R_3$ représentent indépendamment l'un de l'autre l'hydrogène, un groupe méthyle ou le chlore, $R_2$ représente l'hydrogène, un groupe méthyle ou hydroxyle, et $R_4$ représente l'hydrogène ou un groupe méthyle, et ses sels avec des bases physiologiquement acceptables.

7. Acide carboxylique de formule II selon la revendication 1, dans lequel $R_1$, $R_2$, $R_3$, $R_4$ représentent l'hydrogène ou $R_1$, $R_2$, $R_4$ représentent l'hydrogène et $R_3$ représente un groupe méthyle ou $R_1$, $R_4$ représentent l'hydrogène et $R_2$, $R_3$ représentent un groupe méthyle.

8. Acide carboxylique de formule II selon la revendication 1, dans lequel $R_1$, $R_2$, $R_4$ représentent l'hydrogène et $R_3$ est le chlore.

9. Acide carboxylique de formule II selon la revendication 1, dans lequel $R_1$, $R_3$, $R_4$ représentent l'hydrogène et $R_2$ est un groupe hydroxyle.

10. Sel de sodium d'un acide carboxylique selon l'une quelconque des revendications 1 à 9.

11. Procédé de préparation d'acides carboxyliques selon la revendication 1, caractérisé en ce qu'on fait réagir un salicyl-thioamide avec un composé α-halogénocarbonyle de formule

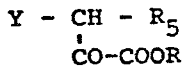

dans laquelle Y représente le chlore ou le brome, $R_5$ représente l'hydrogène, un groupe méthyle, phényle ou —COOR et R représente l'hydrogène ou un groupe alcoyle, ou avec la cystéine ou un sel ou un ester de cystéine.

12. Procédé de préparation d'un acide carboxylique de formule II selon la revendication 1, caractérisé en ce qu'on fait réagir un composé de formule

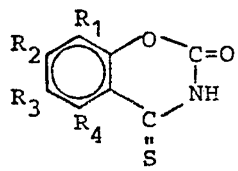

avec la cystéine ou un de ses sels.

13. Médicament contenant un acide carboxylique selon l'une quelconque des revendications 1 à 10, ou ses sels.

14. Composé selon l'une quelconque des revendications 1 à 10, pour utilisation comme médicament pour agir sur le métabolisme du collagène.

15. Acide carboxylique de formule III

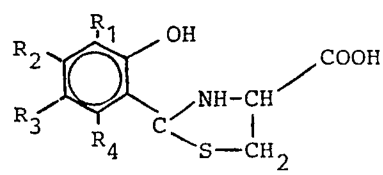

(III)

dans laquelle $R_1$, $R_3$ et $R_4$ représentent l'hydrogène, un groupe méthyle, le chlore ou le brome et $R_1$ ou $R_3$ peuvent être en outre un groupe nitro, et $R_2$ représente l'hydrogène, un groupe méthyle ou hydroxyle, ou ses sels avec une base physiologiquement acceptable, comme médicament pour agir sur le métabolisme du collagène.

16. Composé selon l'une quelconque des revendications 1 à 10 et 15 pour utilisation comme médicament pour le traitement des maladies dans lesquelles le métabolisme du collagène est pathologiquement modifié.

**Revendications** (pour l'Etat contractant AT)

1. Procédé de préparation d'acides carboxyliques de formule générale

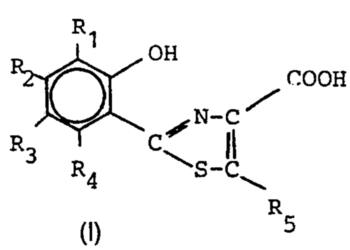

ou

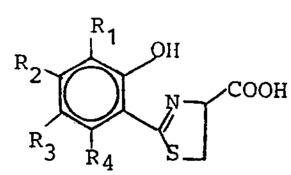

(I) (II)

dans laquelle

$R_1$ et $R_3$ représentent indépendamment l'un de l'autre l'hydrogène, un groupe méthyle, le chlore ou le brome et $R_1$ ou $R_3$ peuvent être en outre un groupe nitro, amino ou sulfamoyl,

$R_2$ représente l'hydrogène, un groupe hydroxyle ou méthyle,

$R_4$ représente l'hydrogène ou un groupe méthyle et

$R_5$ représente l'hydrogène, un groupe méthyle, phényle ou COOH,

l'acide 2-(2-hydroxyphényl)-thiazole-4-carboxylique étant exclus, et leurs sels avec des bases physiologiquement acceptables, caractérisé en ce qu'on fait réagir un salicylthioamide avec un composé α-halogéno-carbonyle de formule

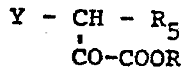

dans laquelle Y représente le chlore ou le brome, $R_5$ représente l'hydrogène, un groupe méthyle, phényle ou —COOr et R représente l'hydrogène ou un groupe alcoyle, ou avec la cystéine ou un sel ou un ester de cystéine et on convertit éventuellement le composé obtenu en un sel.

2. Procédé de préparation d'un acide carboxylique de formule II selon la revendication 1, caractérisé en ce qu'on fait réagir un composé de formule

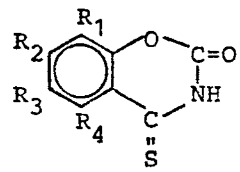

avec la cystéine ou un de ses sels et on convertit éventuellement le composé obtenu en un sel.

3. Procédé selon l'une des revendications 1 ou 2, caractérisé en ce que dans la formule générale I ou II, $R_1$, $R_2$ et $R_4$ représentent l'hydrogène et $R_3$ est un groupe méthyle.

4. Procédé selon l'une des revendications 1 et 2, caractérisé en ce que dans la formule générale I ou II, $R_3$ ou $R_1$ et $R_3$ sont le chlore et les autres R sont l'hydrogène.

5. Procédé selon l'une des revendications 1 ou 2, caractérisé en ce que dans la formule générale I ou II, $R_1$, $R_3$ et $R_4$ représentent l'hydrogène et $R_2$ est un groupe hydroxyle.

6. Procédé selon l'une des revendications 1 et 2, caractérisé en ce que dans la formule générale I ou II, $R_1$ et $R_2$ représentent l'hydrogène, $R_3$ est un groupe méthyle et $R_5$ est un groupe phényle ou COOH.

7. Procédé selon l'une des revendications 1 ou 2, caractérisé en ce que dans la formule générale II, $R_1$ et $R_3$ représentent indépendamment l'un de l'autre l'hydrogène, un groupe méthyle ou le chlore, $R_2$ représente l'hydrogène, un groupe méthyle ou hydroxyle et $R_4$ représente l'hydrogène ou un groupe méthyle.

8. Procédé selon l'une des revendications 1 ou 2, caractérisé en ce que dans la formule générale II, $R_1$, $R_2$, $R_3$, $R_4$ représentent l'hydrogène ou $R_1$, $R_2$, $R_4$ représentent l'hydrogène et $R_3$ est un groupe méthyle ou $R_1$, $R_4$ représentent l'hydrogène et $R_2$, $R_3$ représentent un groupe méthyle.

9. Procédé selon l'une des revendications 1 ou 2, caractérisé en ce que dans la formule générale II, $R_1$, $R_2$, $R_4$ représentent l'hydrogène et $R_3$ est le chlore.

10. Procédé selon l'une des revendications 1 ou 2, caractérisé en ce que dans la formule générale II, $R_1$, $R_3$, $R_4$ représentent l'hydrogène et $R_2$ est un groupe hydroxyle.

11. Procédé selon l'une quelconque des revendications 1 à 10, pour la préparation d'un composé pour utilisation comme médicament pour agir sur le métabolisme du collagène.

12. Procédé pour la préparation d'un composé de formule III

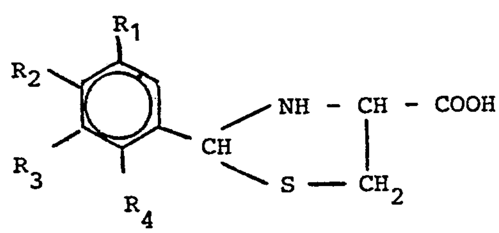

(III)

caractérisé en ce que $R_1$, $R_3$ et $R_4$ représentent l'hydrogène, un groupe méthyle, le chlore ou le brome, et $R_1$ ou $R_3$ peuvent en outre représenter un groupe nitro et $R_2$ représente l'hydrogène, un groupe méthyle ou hydroxyle, pour utilisation comme médicament pour agir sur le métabolisme du collagène, caractérisé en ce qu'on fait réagir un composé de formule

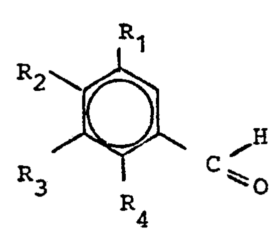

dans laquelle les radicaux $R_1$ à $R_4$ ont les significations définies ci-dessus, avec la cystéine.